# EUROPÄISCHE PATENTSCHRIFT

(11) **EP 0 957 886 B1**
(45) Veröffentlichungstag und Bekanntmachung des Hinweises auf die Patenterteilung: **02.01.2003**
(21) Anmeldenummer: 97911159.8
(22) Anmeldetag: 29.09.1997
(51) Int. Cl.: A61K 7/00, A61K 7/42

(54) **SONNENSCHUTZMITTEL IN FORM VON O/W-MIKROEMULSIONEN**
SUN SCREENING AGENTS IN THE FORM OF OIL/WATER MICRO EMULSIONS
AGENTS DE PROTECTION CONTRE LE SOLEIL, SOUS FORME DE MICRO-EMULSIONS DU TYPE HUILE DANS L'EAU

(30) Priorität: 07.10.1996 DE 19641274
(43) Veröffentlichungstag der Anmeldung: 24.11.1999
(73) Patentinhaber: Cognis Deutschland GmbH & Co. KG, 40589 Düsseldorf (DE)
(72) Erfinder: SEIPEL, Werner, D-40723 Hilden (DE); FABRY, Bernd, D-41352 Korschenbroich (DE); KAHRE, Jörg, D-42799 Leichlingen (DE)
(74) Vertreter: Fabry, Bernd, Dr.
(86) Internationale Anmeldenummer: EP9705349
(87) Internationale Veröffentlichungsnummer: WO98015253

(56) Entgegenhaltungen:
- DE-U- 29 520 749

## Beschreibung

### Gebiet der Erfindung

Die Erfindung betrifft Sonnenschutzmittel in Form von O/W-Mikroemulsionen, enthaltend Ölkörper, ausgewählte anionische Emulgatoren und UV-Lichtschutzfilter sowie die Verwendung dieser Mischungen zur Herstellung von Sonnenschutzmitteln.

### Stand der Technik

Die Pigmentierung normaler Haut führt unter dem Einfluß von Sonnenstrahlung zur Bildung von Melaninen. Dabei ruft die Bestrahlung mit langwelligem UV-A Licht die Dunkelung der in der Epidermis bereits vorhandenen Melaninkörper hervor, ohne daß schädigende Folgen zu erkennen sind, während die kurzwellige UV-B Strahlung die Bildung neuen Melanins bewirkt. Ehe das schützende Pigment jedoch gebildet werden kann, unterliegt die Haut der Einwirkung der ungefilterten Strahlung, die je nach Expositionsdauer zu Hautrötungen (Erythemen), Hautentzündungen (Sonnenbrand) oder gar Brandblasen führen kann. Die mit derartigen Hautläsionen verbundenen Belastungen des Organismus, beispielsweise im Zusammenhang mit der Ausschüttung von Histaminen, kann zusätzlich zu Kopfschmerzen, Mattigkeit, Fieber, Herz- und Kreislaufstörungen und dergleichen führen. Für den Verbraucher, der sich vor den schädlichen Aspekten der Sonneneinstrahlung schützen will, bietet der Markt eine Vielzahl von Produkten, bei denen es sich ganz überwiegend um Öle und milchige Emulsionen handelt, die neben einigen Pflegestoffen vor allem UV-Lichtschutzfilter enthalten. Übersichten hierzu finden sich beispielsweise von P.Finkel in **Parf.Kosm. 76, 432 (1995)** und S.Schauder in **Parf.Kosm. 76, 490 (1995).**

Dennoch besteht im Markt weiterhin das Bedürfnis nach Produkten mit einem verbesserten Leistungsspektrum. Besonderes Interesse gilt dabei Zubereitungen, die die Einarbeitung von größeren Mengen UV-Lichtschutzfiltern erlauben, ohne daß im Laufe der Lagerung eine Phasentrennung bzw. eine Sedimentation stattfindet. Eine nach der Phaseninversionstemperaturmethode hergestellte Formulierung, wie beispielsweise in der Europäischen Patentanmeldung **EP-A1 0 667 144** (L'Oreal) beschrieben, neigt bei der Einarbeitung von größeren Mengen Titandioxid sehr rasch zur Ausscheidung des dispergierten Feststoffes. Ein weiteres Problem besteht darin, daß viele UV-Lichtschutzfilter mit den weiteren Bestandteilen der Rezeptur in Wechselwirkung treten können, was zu einer chemische Reaktion und ebenfalls zu einer Abnahme der Lagerbeständigkeit führt. Schließlich wünscht der Verbraucher transparente Formulierungen, die auch gegenüber sehr empfindlicher Haut eine hohe hautkosmetische Verträglichkeit aufweisen. Die komplexe Aufgabe der Erfindung hat somit darin bestanden, Sonnenschutzmittel zur Verfügung zu stellen, die sich gleichzeitig durch besondere Phasenstabilität, Lagerbeständigkeit, Transparenz und Verhäglichkeit gegenüber empfindlicher Haut auszeichnen.

### Beschreibung der Erfindung

Gegenstand der Erfindung sind Sonnenschutzmittel in Form von O/W-Mikroemulsionen, enthaltend
(a) Ölkörper,
(b) Monoglycerid(ether)sulfate und
(c) UV-Lichtschutzfilter.

Überraschenderweise wurde gefunden, daß O/W-Mikroemulsionen der geschilderten Art selbst bei Einarbeitung größerer Mengen UV-Lichtschutzmittel äußerst lagerbeständig sind und sich durch eine besonders hohe hautkosmetische Verträglichkeit auszeichnen. Aufgrund ihrer Feinteiligkeit sind sie zudem transparent, was zum ästhetischen Erscheinungsbild der Produkte beiträgt. Die Erfindung schließt die Erkenntnis ein, daß besonders vorteilhafte Zubereitungen erhalten werden, wenn man als Emulgatoren Mischungen von (b1) Monoglyceridsulfaten und (b2) Alkylethersulfaten, Alkyloligoglucosiden und/oder Fettsäure-N-alkylglucamiden im Gewichtsverhältnis 10 : 90 bis 90 : 10 und vorzugsweise 25 : 75 bis 75 : 25 einsetzt

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. naphthenische Kohlenwasserstoffe in Betracht. Als Ölkörper können ferner auch Siliconverbindungen eingesetzt weiden, beispielsweise Dimethylpolysiloxane, Methylphenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Die Ölkörper können in den erfindungsgemäßen Mitteln in Mengen von 1 bis 90, vorzugsweise 5 bis 75 und insbesondere 10 bis 50 Gew.-% - bezogen auf den nicht-wäßrigen Anteil - enthalten sein.

### Monoglycerid(ether)sulfate

Monoglyceridsulfate und Monoglyceridethersulfate stellen bekannte anionische Tenside dar, die nach den einschlägigen Methoden der präparativen organischen Chemie erhalten werden können. Üblicherweise geht man zu ihrer Herstellung von Triglyceriden aus, die gegebenenfalls nach Ethoxylierung zu den Monoglyceriden umgeestert und nachfolgend sulfatiert und neutralisiert werden. Gleichfalls ist es möglich, die Partialglyceride mit geeigneten Sulfatierungsmitteln, vorzugsweise gasförmiges Schwefeltrioxid oder Chlorsulfonsäure umzusetzen [vgl. **EP-B1 0 561 825, EP-B1 0 561 999** (Henkel)]. Die neutralisierten Stoffe können - falls gewünscht - einer Ultrafiltration unterworfen werden, um den Elektrolytgehalt auf ein gewünschtes Maß zu vermindern **[DE-A1 42 04 700** (Henkel)]. Übersichten zur Chemie der Monoglyceridsulfate sind beispielsweise von A.K.Biswas et al. in **J.Am.Oil.Chem.Soc. 37**, **171 (1960)** und F.U.Ahmed **J.Am.Oil.Chem.Soc. 67, 8 (1990)** erschienen.

Die im Sinne der Erfindung einzusetzenden Monoglycerid(ether)sulfate folgen der Formel (I) 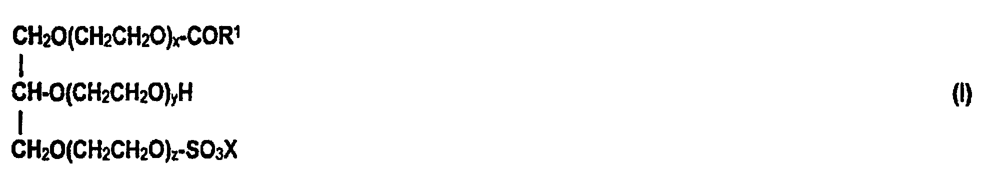 in der R¹CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (I) eingesetzt, in der R¹CO für einen linearen Acylrest mit 8 bis 18 Kohlenstoffatomen steht. Der Anteil der Monoglycerid(ether)sulfate liegt üblicherweise - bezogen auf den nicht-wäßrigen Teil - bei 10 bis 90, vorzugsweise 25 bis 75 und insbesondere 40 bis 60 Gew.-%.

### Alkylethersulfate

Alkylethersulfate ("Ethersulfate"), die als anionische Co-Emulgatoren in Betracht kommen, stellen bekannte Tenside dar, die großtechnisch durch SO₃- oder Chlorsulfonsäure (CSA)-Sulfatierung von Fettalkohol- oder Oxoalkoholpolyglycolethem und nachfolgende Neutralisation hergestellt werden. Im Sinne der Erfindung kommen Ethersulfate in Betracht, die der Formel (II) folgen,

**R**^{**2**}**O-(CH**_{**2**}**CH**_{**2**}**O)**_{**m**}**SO**_{**3**}**X** **(II)**

in der R² für einen linearen oder verzweigten Alkyl- und/oder Alkenylrest mit 6 bis 22 Kohlenstoffatomen,m für Zahlen von 1 bis 10 und X für ein Alkali- und/oder Erdalkalimetall, Ammonium, Alkylammonium, Alkanolammonium oder Glucammonium steht. Typische Beispiele sind die Sulfate von Anlagerungsprodukten von durchschnittlich 1 bis 10 und insbesondere 2 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, in Form ihrer Natrium- und/oder Magnesiumsalze. Die Ethersulfate können dabei sowohl eine konventionelle als auch eine eingeengte Homologenverteilung aufweisen. Besonders bevorzugt ist der Einsatz von Ethersulfaten auf Basis von Addukten von durchschnittlich 2 bis 3 Mol Ethylenoxid an technische C_{12/14}- bzw. C_{12/18}- Kokosfettalkoholfraktionen in Form ihrer Natrium- und/oder Magnesiumsalze.

### Alkyl- und/oder Alkenyloligoglykoside

Alkyl- und Alkenyloligoglykoside, die als nichtionische Co-Emulgatoren in Betracht kommen, stellen bekannte Tenside dar, die der Formel (III) folgen,

**R**^{**3**}**O-[G]**_{**p**} **(III)**

in der R³ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP-A1 0 301 298** und **WO 90/03977** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkylund/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R³ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R³ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Fettsäure-N-alkylpolyhydroxyalkylamide

Fettsäure-N-alkylpolyhydroxyalkylamide, die als nichtionische Co-Emulgatoren ebenfalls in Betracht kommen, stellen bekannte Tenside dar, die der Formel (IV) folgen, 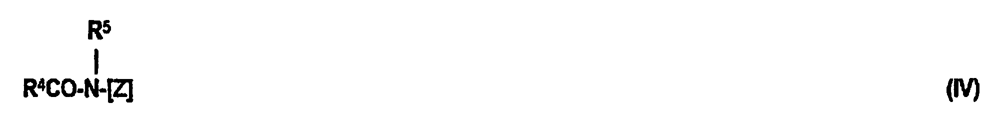 in der R⁴CO für einen aliphatischen Acylrest mit 6 bis 22 Kohlenstoffatomen, R⁵ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanofamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H.Kelkenberg findet sich in Tens.Surf. **Deterg. 25, 8 (1988).**

Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Faltsäure-N-alkylglucamide** dar, wie sie durch die Formel (V) wiedergegeben werden:

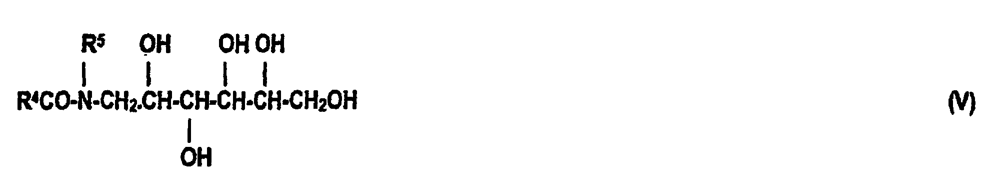

Vorzugsweise werden als Fettsäure-N-alkylpolyhydroxyalkylamide Glucamide der Formel (V) eingesetzt, in der R⁶ für eine Alkylgruppe steht und R⁴CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkyl-glucamide der Formel (V), die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

Der Anteil der genannten Co-Emulgatoren liegt in Summe üblicherweise - bezogen auf den nicht-wäßrigen Teil - bei 5 bis 75, vorzugsweise 10 bis 50 und insbesondere 15 bis 30 Gew.-%.

### UV-Lichtschutzfilter

Unter UV-Lichtschutzfiltern sind organische Substanzen zu verstehen, die in der Lage sind, ultraviolette Strahlen zu absorbieren und die aufgenommene Energie in Form längerwelliger Strahlung, z.B. Wärme wieder abzugeben. Eine Übersicht hierzu findet sich beispielsweise in **Parf.Kosm. 74, 485 (1993).** Typische Beispiele sind 4-Aminobenzoesäure sowie ihre Ester und Derivate (z.B. 2-Ethylhexyl-pdimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxy-benzophenon), Dibenzoylmethane, Salicylatester, 2-Phenylbenzimadozol-5-sulfonsäure, 1-(4-tert.Butylphenyl)-3-(4'-methoxyphenyl)-propan-1,3-dion, 3-(4'-Methyl)benzyliden-boman-2-on, Methylbenzylidencampher und dergleichen.

Die erfindungsgemäßen Mittel können als Lichtschutzfilter des weiteren auch feindisperse Metalloxide bzw. Salze enthalten. Typische Beispiele sind Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen.

Neben den beiden vorgenannten Gruppen primärer Lichtschutzstoffe können die erfindungsgemäßen Mittel ferner auch sekundäre Lichtschutzmittel vom Typ der Antioxidantien enthalten, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Der Anteil der Lichtschutzmittel an den erfindungsgemäßen Mitteln liegt - bezogen auf den nicht-wäßrigen Anteil - üblicherweise bei 10 bis 90, vorzugsweise 25 bis 75 und insbesondere 40 bis 60 Gew.-%. Die erfindungsgemäßen Mittel als solche können 1 bis 95, vorzugsweise 5 bis 80 und insbesondere 10 bis 60 Gew.-% Wasser enthalten. Werden als Lichtschutzmittel organische Verbindungen eingesetzt, können zur Herstellung der Zubereitungen deren co-emulgierenden Eigenschaften mitgenutzt werden.

### Mikroemulsionen

Mikroemulsionen stellen optisch isotrope, thermodynamisch stabile Systeme dar, die Ölkomponenten, Emulgatoren und Wasser enthalten. Das klare bzw. transparente Aussehen der Mikroemulsionen ist eine Folge der geringen Teilchengröße der dispergierten Emulsionströpfchen, die im wesentlichen unter 300, vorzugsweise bei 50 bis 300 nm liegen. Im Bereich zwischen 100 und 300 nm werden dabei feinteilige, in der Durchsicht braunrote und im Auflicht bläulich schimmernde Mikroemulsionen erhalten. Im Bereich unterhalb eines Tröpfchendurchmessers von 100 nm sind Mikroemulsionen klar. Die Herstellung der Mittel erfolgt vorzugsweise in einem Kaltverfahren, bei dem man Ölkörper und Emulgatoren mit Wasser emulgiert und anschließend die UV-Lichtschutzfilter sowie gegebenenfalls weitere Zusatzstoffe hinzugibt. Durch Zusatz von Wasser und/oder Hydrotropen kann der gewünschte Aktivsubstanzgehalt eingestellt werden.

### Gewerbliche Anwendbarkeit

Die erfindungsgemäßen Mittel zeichnen sich durch eine hohe Transparenz und Phasenstabilität bei besonders vorteilhafter hautkosmetischer Verträglichkeit aus. Typische Zubereitungen haben die folgende Zusammensetzung:
(a) 1 bis 90, vorzugsweise 5 bis 80 Gew.-% Ölkörper,
(b) 10 bis 90, vorzugsweise 25 bis 75 Gew.-% Emulgatoren und
(c) 10 bis 90, vorzugsweise 25 bis 75 Gew.-% UV-Lichtschutzfilter,
mit der Maßgabe, daß sich die Mengenangaben zu 100 Gew.-% ergänzen. Innerhalb der genannten Konzentrationsbereiche werden besonders stabile und feinteilige Mikroemulsionen erhalten.

Als weitere Inhaltsstoffe können sie in untergeordneten Mengen weitere, mit den anderen Inhaltsstoffen kompatible Aniontenside enthalten. Typische Beispiele sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Glycerinethersulfate, Hydroxymischethersulfate, Fettsäureamid(ether)sulfate, Mono- und Dialkylsulfosuccinate, Mono- und Dialkyl-sulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekonden-sate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Die erfindungsgemäßen Mittel können ferner als weitere Hilfs- und Zusatzstoffe Co-Emulgatoren, Überfettungsmittel, Stabilisatoren, Wachse, Konsistenzgeber, Verdickungsmittel, Kationpolymere, biogene Wirkstoffe, Konservierungsmittel, Hydrotrope, Solubilisatoren, Farb- und Duftstoffe enthalten.

Als **Co-Emulgatoren** kommen beispielsweise nichtionogene Tenside aus mindestens einer der folgenden Gruppen in Frage:
(b1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Anlagerungsprodukte von 15 bis 60 Mol Ethylenoxid an Ricinusöl und/oder gehärtetes Ricinusöl;
(b5) Polyol- und insbesondere Polyglycerinester wie z.B. Polyglycerinpolyricinoleat oder Potyglycerinpoly-12-hydroxystearat. Ebenfalls geeignet sind Gemische von Verbindungen aus mehreren dieser Substanzklassen;
(b6) Anlagerungsprodukte von 2 bis 15 Mol Ethylenoxid an Ricinusöl und/oder gehärtetes Ricinusöl;
(b7) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b8) Trialkylphosphate;
(b9) Wollwachsalkohole;
(b10) Polysiloxan-Polyalkyl-Polyether-Copolymere bzw. entsprechende Derivate;
(b11) Mischester aus Pentaerythrit, Fettsäuren, Citronensäure und Fettalkohol gemäß **DE-PS 11 65 574** sowie
(b12) Polyalkylenglycole.

Die Anlagerungsprodukte von Ethylenoxid und/oder von Propylenoxid an Fettalkohole; Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid undl oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquatemierte Difettsäuretriethanolaminester-Salze, besonders bevorzugt sind.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als Konsistenzgeber kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden undloder Fettsäure-N-methyl-glucamiden gleicher Kettenlänge undloder Polyglycerinpoly-12-hydroxystearaten, Geeignete Verdickungsmittel sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise ethoxylierte Pettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete kationische Polymere sind beispielsweise kationische Cellulosederivate, kationische Stärke, Copolymere von Diallylammoniumsalzen und Acrylamiden, quatemierte Vinylpyrrolidon/Vinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Coming, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jagua® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Typische Beispiele für Fette sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Als **Perlglanzwachse** können insbesondere Mono- und Difettsäuieester von Polyalkylenglycolen, Partialglyceride oder Ester von Fettalkoholen mit mehrwertigen Carbonsäuren bzw.

Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Zur Verbesserung des Fließverhaltens können ferner **Hydrotrope** wie beispielsweise Ethanol, Isopropylalkohol, Propylenglycol oder Glucose eingesetzt werden. Als **Konservierungsmittel** eignen sich beispielsweise Phenoxyethanol, Formaldehydlösung, Parabene, Pentandiol oder Sorbinsäure. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen.Ein weiterer Gegenstand der Erfindung betrifft schließlich die Verwendung der erfindungsgemäßen O/W-Mikroemulsionen zur Herstellung von Sonnenschutzmitteln.

### Beispiele

Die erfindungsgemäßen O/W-Mikroemulsionen R1 bis R6 wurden in einem Kaltverfahren hergestellt. Dazu wurden die Ölkörper zusammen mit den öllöslichen bzw. öldispergierbaren UV-Lichtschutzfiltern und den Emulgatoren in Wasser emulgiert; wachsartige Komponenten wurden aufgeschmolzen und bei leicht erhöhter Temperatur eingearbeitet, wasserlösliche bzw. wasserdispergierbare UV-Filter zusammen mit der wäßrigen Phase eingebracht. Es wurden wasserklare Mikroemulsionen erhalten. Die Transmission der Emulsionen wurde photometrisch bei 650 nm bestimmt. Die Beurteilung der Stabilität erfolgte nach einer Lagerung von 12 Wochen bei 40 °C. Hierbei bedeuten (+++) = keine Phasentrennung/Sedimentation und (++) keine Phasentrennung/geringfügige Trübung. Die Ergebnisse sind In Tabelle 1 zusammengefaßt.

**Tabelle 1**

| O/W-Mikroemulsionen | | | | | | | |
|---|---|---|---|---|---|---|---|
| **Komponente** | **INCI-Bezeichnung** | **R1** | **R2** | **R3** | **R4** | **R5** | **R6** |
| Plantapon CMGS | Sodium Coco Monoglycerol Sulfate | 12,0 | 6,0 | 6,0 | 6,0 | 6,0 | 3,0 |
| Texapon N 70 | Sodium Laureth Sulfate | | 6,0 | | 3,0 | 2,0 | 4,0 |
| Plantacare© 1200 | Dodecyl Polyglucose | | | 6,0 | 3,0 | 4,0 | 5,0 |
| Monomuis® 90-O 18 | Glyceryl Oleate | 6,0 | - | - | - | - | - |
| Eutanol® G | Octyldodecanol | 3,0 | 15,0 | - | - | - | 15,0 |
| Cetiol® OE | Dicaprilyl Ether | - | 15,0 | - | - | - | 20,0 |
| Cetiol® V | Decyl Oleate | - | - | 35,0 | - | - | - |
| Myritol® 318 | Caprylic/Capric Triglyceride | - | - | - | 35,0 | - | - |
| Cetiol® SN | Cetearyl Isononanoate | - | - | - | - | 35,0 | - |
| Parsol® MCX | Methoxyzimtsäure-2-ehylhexylester | 5,0 | 9,0 | - | - | - | - |
| | Methylbenzylidencampher | - | - | 5,0 | - | - | - |
| Uvinul® T 150 | Octyl Triazone | - | - | - | 5,0 | - | - |
| Titandioxid | | - | - | - | - | 5,0 | - |
| Copherol® F 1300 | Tocopherol | - | - | - | - | - | 10,0 |
| Wasser | | ad 100 | | | | | |
| ***Lagerstabilität*** | | ++ | ++ | ++ | ++ | +++ | +++ |
| ***Transmission [%]*** | ― | 92 | 90 | 90 | 92 | 95 | 95 |

## Patentansprüche

1. Sonnenschutzmittel in Form von O/W-Mikroemulsionen, enthaltend
(a) Ölkörper,
(b) Monoglycerid(ether)sulfate und
(c) UV-Lichtschutzfilter.

2. Mittel nach Anspruch 1, **dadurch gekennzeichnet, daß** sie Ölkörper enthalten, die ausgewählt sind aus der Gruppe, die gebildet wird von Guerbetalkoholen auf Basis von Fettalkoholen mit 6 bis 18, vorzugsweise 8 bis 10 Kohlenstoffatomen, Estem von linearen C₆-C₂₀-Fettsäuren mit linearen C₆-C₂₀-Fettalkoholen, Estern von verzweigten C₆-C₁₃-Carbonsäuren mit linearen C₆-C₂₀-Fettalkoholen, Estern von linearen C₆-C₁₈-Fettsäuren mit verzweigten Alkoholen, Estem von linearen undl oder verzweigten Fettsäuren mit mehrwertigen Alkoholen und/oder Guerbetalkoholen, Triglyceriden auf Basis C₆-C₁₀-Fettsäuren, pflanzlichen Ölen, verzweigten primären Alkoholen, substituierten Cyclohexanen, Guerbetcarbonaten, Dialkylethem und/oder aliphatischen bzw. naphthenischen Kohlenwasserstoffen sowie Siliconverbindungen.

3. Mittel nach den Ansprüchen 1 und 2, **dadurch gekennzeichnet, daß** sie als Emulgatorkomponente Monoglycerid(ether)sulfate der Formel (I) enthalten, 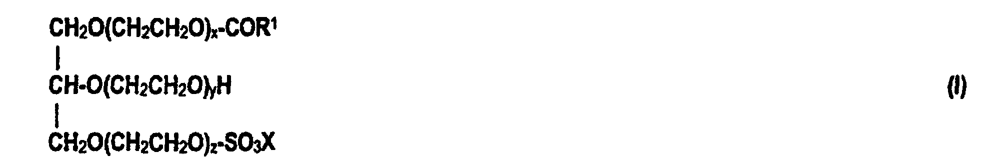 in der R¹CO für einen linearen oder verzweigten Acylrest mit 6 bis 22 Kohlenstoffatomen, x, y und z in Summe für 0 oder für Zahlen von 1 bis 30 und X für ein Alkali- oder Erdalkalimetall steht.

4. Mittel nach den Ansprüchen 1 bis 3, **dadurch gekennzeichnet, daß** sie als Emulgatorkomponente weiterhin Alkylethersulfate der Formel (II) enthalten,
**R**^{**2**}**O-(CH**_{**2**}**CH**_{**2**}**O)**_{**m**}**SO**_{**3**}**X** **(II)**
in der R² für einen linearen oder verzweigten Alkyl- und/oder Alkenylrest mit 6 bis 22 Kohlenstoffatomen, m für Zahlen von 1 bis 10 und X für ein Alkali- und/oder Erdalkalimetall, Ammonium, Alkylammonium, Alkanotammonium oder Glucammonium steht.

5. Mittel nach den Ansprüchen 1 bis 4, **dadurch gekennzeichnet, daß** sie als Emulgatorkomponente weiterhin Alkyl- und Alkenyloligoglykoside der Formel (III) enthalten,
**R**^{**3**}**O-[G]**_{**p**} **(III)**
in der R³ für einen Alkyl- undloder Alkenylrest mit 4 bis 22 Kohlenstoffatomen, G für einen Zuckerrest mit 5 oder 6 Kohlenstoffatomen und p für Zahlen von 1 bis 10 steht.

6. Mittel nach den Ansprüchen 1 bis 5, **dadurch gekennzeichnet, daß** sie als Emulgatorkomponente weiterhin Fettsäure-N-alkylpolyhydroxyalkylamide der Formel (IV) enthalten, 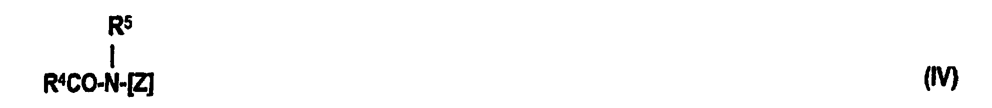 in der R⁴CO für einen aliphatischen Acylrest mit 6 bis 22 Kohlenstoffatomen, R⁵ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

7. Mittel nach den Ansprüchen 1 bis 6, **dadurch gekennzeichnet, daß** sie UV-Lichtschutzfilter enthalten, die ausgewählt sind aus der Gruppe, die gebildet wird von 4-Aminobenzoesäure sowie ihren Estem und Derivaten, Methoxyzimtsäure und ihren Derivaten, Benzophenonen, Dibenzoylmethanen, Salicylatestern, 2-Phenylbenzimadozol-5-sulfonsäure, 1-(4-tert.Butylphenyl)-3-(4'methoxyphenyl)-propan-1,3-dion, 3-(4'-Methyl)benzyliden-boman-2-on und Methylbenzylidencampher.

8. Mittel nach den Ansprüchen 1 bis 7, **dadurch gekennzeichnet, daß** sie als UV-Lichtschutzfilter feindisperse Metalloxide bzw. Salze enthalten, die ausgewählt sind aus der Gruppe, die gebildet wird von Titantoxid, Zinkoxid, Eisenoxid, Aluminiumoxid, Ceroxid, Zirkoniumoxid, Silicate (Talk) und Bariumsulfat.

9. Mittel nach den Ansprüchen 1 bis 8, **dadurch gekennzeichnet, daß** sie als UV-Lichtschutzmittel Antioxidantien enthalten, die ausgewählt sind aus der Gruppe, die gebildet wird von Superoxid-Dismutase, Tocopherolen (Vitamin E) und Ascorbinsäure (Vitamin C).

10. Verwendung von O/W-Mikroemulsionen nach Anspruch 1 zur Herstellung von Sonnenschutzmitteln.

## Claims

1. Sun protection compositions in the form of o/w microemulsions containing
(a) oils,
(b) monoglyceride (ether)sulfates and
(c) UV filters.

2. Compositions as claimed in claim 1, **characterized in that** they contain oils selected from the group consisting of Guerbet alcohols based on fatty alcohols containing 6 to 18 and preferably 8 to 10 carbon atoms, esters of linear C₆₋₂₀ fatty acids with linear C₆₋₂₀ fatty alcohols, esters of branched C₆₋₁₃ carboxylic acids with linear C₆₋₂₀ fatty alcohols, esters of linear C₆₋₁₈ fatty acids with branched alcohols, esters of linear and/or branched fatty acids with polyhydric alcohols and/or Guerbet alcohols, triglycerides based on C₆₋₁₀ fatty acids, vegetable oils, branched primary alcohols, substituted cyclohexanes, Guerbet carbonates, dialkyl ethers and/or aliphatic or naphthenic hydrocarbons and silicone compounds.

3. Compositions as claimed in claims 1 and 2, **characterized in that** they contain monoglyceride (ether) sulfates corresponding to formula **(I)**: 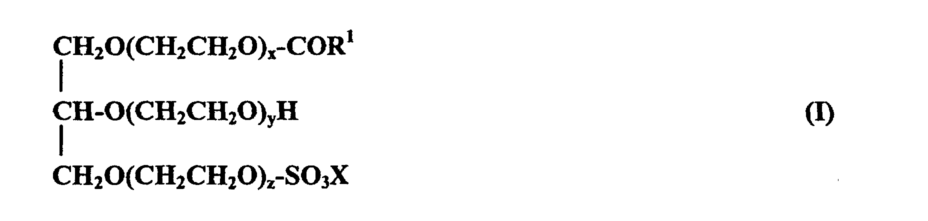 in which R¹CO is a linear or branched acyl group containing 6 to 22 carbon atoms, x, y and z together stand for 0 or for numbers of 1 to 30 and X is an alkali metal or alkaline earth metal,
as emulsifier component.

4. Compositions as claimed in claims 1 to 3, **characterized in that** they additionally contain alkyl ether sulfates corresponding to formula **(II):**
**R**^{**2**}**O-(CH**_{**2**}**CH**_{**2**}**O)**_{**m**}**SO**_{**3**}**X** **(II)**
in which R² is a linear or branched alkyl and/or alkenyl radical containing 6 to 22 carbon atoms, m is a number of 1 to 10 and X is an alkali metal and/or alkaline earth metal, ammonium, alkylammonium, alkanolammonium or glucammonium,
as emulsifier component.

5. Compositions as claimed in claims 1 to 4, **characterized in that** they additionally contain alkyl and alkenyl oligoglycosides corresponding to formula **(III)**:
**R**^{**3**} **O-[G]**_{**p**} **(III)**
in which R³ is an alkyl and/or alkenyl radical containing 4 to 22 carbon atoms, G is a sugar unit containing 5 or 6 carbon atoms and p is a number of 1 to 10, as emulsifier component.

6. Compositions as claimed in claims 1 to 5, **characterized in that** they additionally contain fatty acid N-alkyl polyhydroxyalkylamides corresponding to formula **(IV):** 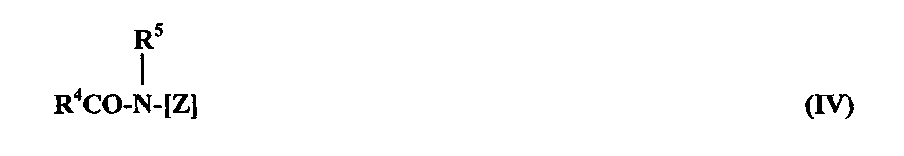 where R⁴CO is an aliphatic acyl radical containing 6 to 22 carbon atoms, R⁵ is hydrogen, an alkyl or hydroxyalkyl radical containing 1 to 4 carbon atoms and [Z] is a linear or branched polyhydroxyalkyl radical containing 3 to 12 carbon atoms and 3 to 10 hydroxyl groups, as emulsifier component.

7. Compositions as claimed in claims 1 to 6, **characterized in that** they contain UV filters selected from the group consisting of 4-aminobenzoic acid and esters and derivatives thereof, methoxycinnamic acid and derivatives thereof, benzophenones, dibenzoyl methanes, salicylate esters, 2-phenyl benzimidazole-5-sulfonic acid, 1-(4-tert.butylphenyl)-3-(4'-methoxyphenyl)-propane-1,3-dione, 3-(4'-methyl)-benzylidene bornan-2-one and benzylidene camphor.

8. Compositions as claimed in claims 1 to 7, **characterized in that** they contain finely dispersed metal oxides or salts selected from the group consisting of titanium oxide, zinc oxide, iron oxide, aluminium oxide, serium oxide, zirconium oxide, silicates (talcum) and barium sulfate as UV filters.

9. Compositions as claimed in claims 1 to 8, **characterized in that** they contain antioxidants selected from the group consisting of Superoxid-Dismutase, tocopherols (vitamin E) and ascorbic acid (vitamin C) as UV filters.

10. The use of the o/w microemulsions claimed in claim 1 for the production of sun protection compositions.

## Revendications

1. Agents de protection solaire sous forme de microémulsions H/E, contenant
(a) des corps huileux,
(b) des (éther)sulfates de monoglycérides et
(c) des filtres de protection contre la lumière UV.

2. Agents selon la revendication 1,
**caractérisés en ce qu'**
ils contiennent des corps huileux choisis dans le groupe formé par les alcools de Guerbet à base d'alcools gras comportant de 6 à 18, de préférence de 8 à 10 atomes de carbone, les esters d'acides gras en C₆ à C₂₀ linéaires avec des alcools gras en C₆ à C₂₀ linéaires, les esters d'acides carboxyliques en C₆ à C₁₃ ramifiés avec des alcools gras en C₆ à C₂₀ linéaires, les esters d'acides gras en C₆ à C₁₈ linéaires avec des alcools ramifiés, les esters d'acides gras linéaires et/ou ramifiés avec des alcools polyvalents et/ou des alcools de Guerbet, les triglycérides à base d'acides gras en C₆ à C₁₀, les huiles végétales, les alcools primaires ramifiés, les cyclohexanes substitués, les carbonates de Guerbet, les dialkyléthers et/ou les hydrocarbures aliphatiques ou selon les cas naphténiques ainsi que les composés de silicones.

3. Agents selon les revendications 1 ou 2,
**caractérisés en ce qu'**
ils contiennent comme composants émulsifiants des (éther)sulfates de monoglycérides de formule (I) 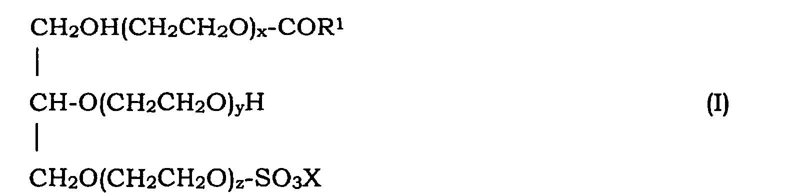 dans laquelle R¹CO représente un radical acyle linéaire ou ramifié comportant de 6 à 22 atomes de carbone, x, y et z valent au total 0 ou des nombres allant de 1 à 30 et X représente un métal alcalin ou alcalino-terreux.

4. Agents selon les revendications 1 à 3,
**caractérisés en ce qu'**
ils contiennent en outre comme composants émulsifiants des sulfates d'alkyléthers de formule (II)
R²O-(CH₂CH₂O)ₘSO₃X (II)
dans laquelle R² représente un radical alkyle et/ou alcényle linéaire ou ramifié comportant de 6 à 22 atomes de carbone, m représente des nombres allant de 1 à 10 et X représente un métal alcalin et/ou alcalino-terreux, l'ammonium, un alkylammonium, un alcanolammonium ou un glucammonium.

5. Agents selon les revendications 1 à 4,
**caractérisés en ce qu'**
ils contiennent en outre comme composants émulsifiants des alkyl- et alcényloligoglycosides de formule (III)
R³O-[G]ₚ (III)
dans laquelle R³ représente un radical alkyle et/ou alcényle comportant de 4 à 22 atomes de carbone, G représente un radical sucré comportant 5 ou 6 atomes de carbone et p représente des nombres allant de 1 à 10.

6. Agents selon les revendications 1 à 5,
**caractérisés en ce qu'**
ils contiennent en outre comme composants émulsifiants des N-alkylpolyhydroxyalkylamides d'acides gras de formule (IV) 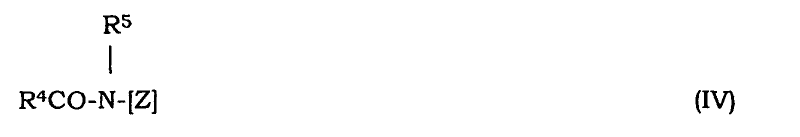 dans laquelle R⁴CO représente un radical acyle aliphatique comportant de 6 à 22 atomes de carbone, R⁵ représente un hydrogène, un radical allyle ou hydroxyalkyle comportant de 1 à 4 atomes de carbone et [Z] représente un radical polyhydroxyalkyle linéaire ou ramifié comportant de 3 à 12 atomes de carbone et de 3 à 10 groupes hydroxyle.

7. Agents selon les revendications 1 à 6,
**caractérisés en ce qu'**
ils contiennent des filtres protecteurs contre la lumière UV, qui sont choisis dans le groupe formé par l'acide 4-aminobenzoïque ainsi que ses esters et dérivés, l'acide méthoxycinnamique et ses dérivés, les benzophénones, les dibenzoylméthanes, les esters de salicylates, l'acide 2-phénylbenzimidazole-5-sulfonique, la 1-(4-tert.butylphényl)-3-(4'-méthoxyphényl)-propane-1,3-dione, la 3-(4'-méthyl)benzylidène-bornane-2-one et le méthylbenzylidènecamphre.

8. Agents selon les revendications 1 à 7,
**caractérisés en ce qu'**
ils contiennent comme filtres protecteurs contre la lumière UV des oxydes ou selon les cas des sels métalliques finement dispersés qui sont choisis dans le groupe formé par l'oxyde de titane, l'oxyde de zinc, l'oxyde de fer, l'oxyde d'aluminium, l'oxyde de cérium, l'oxyde de zirconium, les silicates (talc) et le sulfate de baryum.

9. Agents selon les revendications 1 à 8,
**caractérisés en ce qu'**
ils contiennent comme agents de protection contre la lumière UV des antioxydants qui sont choisis dans le groupe formé par la superoxyde dismutase, les tocophérols (vitamine E) et l'acide ascorbique (vitamine C).

10. Utilisation de microémulsions H/E selon la revendication 1 pour la production d'agents de protection solaire.
